# EUROPEAN PATENT APPLICATION

(11) **EP 1 637 146 A2**
(43) Date of publication of application: **22.03.2006**
(21) Application number: 05076853.0
(22) Date of filing: 29.07.1999
(51) Int. Cl.: A61K 35/14, A61K 39/395, C12N 5/06, A61P 37/06

(54) **Ex vivo treatment of allogeneic and xenogeneic T-cells with gp39 antagonists**

(30) Priority: 30.07.1998 US 124683
(62) Divisional of application: 99944997.8
(71) Applicant: REGENTS OF THE UNIVERSITY OF MINNESOTA, Minneapolis, Minnesota 55455 (US); Trustees of Dartmouth College, Hanover, NH 03755 (US)
(72) Inventor: Noelle, Randolph J, Dpt Microbiology&Immunology, Lebanon, NH 03756 (US); Blazar, Bruce R., Golden Valley, MN 55416 (US); Vallera, Daniel A., St Louis Park, MN 55426 (US); Taylor, Patricia A., St. Paul, MN 55104 (US)
(74) Representative: Daniels, Jeffrey Nicholas

(57) **Abstract**

Methods for inducing T-cell non-responsiveness to donor T-cells comprised in transplantation tissues are provided. The methods involve *ex vivo* treatment of donor T-cells with gp39 antagonists.

## Description

### Field of the Invention

Methods of treating transplanted tissue or organs (allogeneic or xenogeneic) *ex vivo* in order to tolerize T-cell contained therein to donor antigens (xenoantigens or alloantigens) are provided. The treated tissue or organ can be transplanted in a recipient with reduced risk of graft-versus-host disease.

### Background of The Invention

To induce antigen-specific T-cell activation and clonal expansion, two signals provided by antigen-presenting cells (APCs) must be delivered to the surface of resting T lymphocytes (Jenkins, M. and Schwartz, R. (1987) *J. Exp. Med.* 165, 302-319; Mueller, D.L., et al. (1990) *J. Immunol.* 144,3701-3709; Williams, LR. and Unanue, E.R. (1990) *J. Immunol.145,* 85-93). The first signal, which confers specificity to the immune response, is mediated via the T-cell receptor (TCR) following recognition of foreign antigenic peptide presented in the context of the major histocompatibility complex (MHC). The second signal, termed co-stimulation, induces T cells to proliferate and become functional (Schwartz, R.H. (1990) *Science* 248,1349-1356). Co-stimulation is neither antigen-specific, nor MHC restricted and is thought to be provided by one or more distinct cell surface molecules expressed by APCs (Jenkins, M.K., et al. (1988) *J. Immunol.* 140, 3324-3330; Linsley, P.S., et al. (1991) *J. Exp. Med.* 173, 721-730; Gimmi, C.D., et al., (1991) *Proc. Natl. Acad. Sci. USA,* 88, 6575-6579; Young, J.W., et al. (1992) *J. Clin. Invest.* 90, 229-237; Koulova, L., et al. (1991)J *Exp. Med.* 173, 759-762; Reiser, H., et al. (1992) *Proc. Natl. Acad. Sci. USA,* 89. 271-275; van-Seventer, G.A., et al. (1990) *J. Immunol.* 144, 4579-4586; LaSalle, J.M., et al., (1991) *J. Immunol.* 147, 774-80; Dustin, M.I., et al., (1989) *J Exp. Med.* 169,503; Armitage, R.J., et al. (1992) *Nature* 357, 80-82; Liu, Y., et al. (1992) *J. Exp. Med*. 175,437-445). One co-stimulatory pathway involved in T cell activation involves the molecule CD28 on the surface of T-cells. This molecule can receive a co-stimulatory signal delivered by a ligand on B-cells or other APCs. Ligands for CD28 include members of the B7 family of B lymphocyte activation antigens such as B7-1 and/or B7-2 (Freedman, A.S. et al. (1987) *J. Immunol.* 137,3260-3267; Freeman, G.J. et al. (1989) *J. Immunol.* 143, 2714-2722, Freeman, G.J. et al. (1991) *J. Exp. Med.* 174, 625-631; Freeman, G.J. et al. (1993) *Science* 262, 909-911; Azuma, M. et al. (1993) *Nature* 366, 76-79; Freeman, G.J. et al. (1993) *J*. *Exp. Med.* 178, 2185-2192). B7-1 and B7-2 are also ligands for another molecule. CTLA4, present on the surface of activated T cells, although the role of CTLA4 in co-stimulation is unclear.

Delivery to a T cell of an antigen-specific signal with a co-stimulatory signal leads to T-cell activation, which can include both T-cell proliferation and cytokine secretion. In contrast, delivery to a T-cell of an antigen-specific signal in the absence of a co-stimulatory signal is thought to induce a state of unresponsiveness or anergy in the T-cell, thereby inducing antigen-specific tolerance in the T-cell.

Interactions between T-cells and B-cells play a central role in immune responses. Induction of humoral immunity to thymus-dependent antigens requires "help" provided by T helper (hereafter Th) cells. While some help provided to B lymphocytes is mediated by soluble molecules released by Th cells (for instance lymphokines such as IL-4 and IL-5), activation of B cells also requires a contact-dependent interaction between B cells and Th cells. Hirohata et al., *J. Immunol*., 140:3736-3744 (1988); Bartlett et al., *J. Immunol.,* 143:1745-1754 (1989). This indicates that B-cell activation involves an obligatory interaction between cell surface molecules on B-cells and Th cells. The molecule(s) on the T-cell therefore mediates contact-dependent helper effector functions of the T-cell. A contact-dependent interaction between molecules on B-cells and T-cells is further supported by the observation that isolated plasma membranes of activated T-cells can provide helper functions necessary for B-cell activation. Brian, *Proc. Natl. Acad. Sci. USA,* 85:564-568 (1988); Hodgkin et al., *J. Immunol.,* 145:2025-2034 (1990); Noelle et al., *J. Immunol.,* 146:1118-1124 (1991).

A molecule, CD40, has been identified on the surface of immature and mature B lymphocytes which, when crosslinked by antibodies, induces B-cell proliferation. Valle et al., *Eur. J. Immunol.,* 19:1463-1467 (1989); Gordon et al., *J. Immunol.,* 140:1425-1430 (1988); Gruber et al., *J. Immunol.,* 142: 4144-4152 (1989). CD40 has been molecularly cloned and characterized. Stamenkovic et al., *EMBO J*., 8:1403-1410 (1989). A ligand for CD40, gp39 (also called CD40 ligand or CD40L and recently CD 154) has also been molecularly cloned and characterized. Armitage et al., *Nature,* 357:80-82 (1992); Lederman et al., *J. Exp. Med.,* 175:1091-1101 (1992); Hollenbaugh et al., *EMBO J*., 11:4313-4319 (1992). The gp39 protein is expressed on activated, but not resting, CD4⁺ Th cells. Spriggs et al., *J. Exp. Med.* 176:1543-1550 (1992); Lane et al., *Eur. J. Immunol.,* 22:2573-2578 (1992); Roy et al., *J*. *Immunol.,* 151:1-14 (1993). Cells transfected with the gp39 gene and expressing the gp39 protein on their surface can trigger B-cell proliferation and, together with other stimulatory signals, can induce antibody production. Armitage et al., *Nature,* 357:80-82 (1992); Hollenbaugh et al., *EMBO J*., 11:4313-4319 (1992).

### Brief Description of the Invention

Graft Versus Host Disease (GVHD) is a multi-organ system destructive process caused by the infusion of donor allogeneic T-cells into recipients. Because acute graft versus host disease occurs in 20-40% of recipients of HLA-identical sibling donor grafts and up to 70-80% of recipients of unrelated donor grafts, approaches to prevent this complication of bone marrow transplantation are needed. Two general type of strategies have been used to date. The first involves the in vivo infusion of immune suppressive agents such as methatrycide, cyclosporine A, and steroids. The acute graft versus host disease instances above are those observed during the infusion of these in vivo immune suppressive agents. In addition to their incomplete protective effects, these immune suppressive agents lead to prolonged periods of immune deficiency after bone marrow transplantation thereby re-exposing the recipient to infectious complications and potentially increasing the incidence of relapse after bone marrow transplantation. A second general approach has involved the ex vivo removal of T-cells from the donor graft. This approach while reasonably effective in preventing acute graft versus host disease results in a higher incidence of graft failure, relapse, infectious complications, and delays immune reconstitution time.

By contrast, the present invention is directed to a method of treating donor T-cells *ex vivo,* to render such T-cells substantially non-responsive to allogeneic or xenogeneic antigens upon transplantation into a host. More specifically, the present invention is directed to a method for treating donor T-cells *ex vivo* with an amount of at least one gp39 (CD154) antagonist and allogeneic or xenogeneic cells or tissues, in order to render such T-cells substantially non-responsive to donor antigens (alloantigens or xenoantigens) upon transplantation into a host containing such allogeneic or xenogeneic cells.

The present invention thus provides an effective means of preventing or inhibiting graft-versus-host disease responses that would otherwise potentially occur upon transplantation of donor T-cells, or tissues or organs containing, e.g., donor bone marrow or peripheral blood cells into a recipient.

Preferably, donor T-cells will be incubated *ex vivo* with a sufficient amount of an anti-gp39 antibody and cells from the transplant recipient, for a sufficient time, to render the donor T-cells substantially non-responsive to recipient cells upon transplantation.

This will generally be accomplished by conducting a mixed lymphocyte reaction *in vitro* using donor T-cells and irradiated T-cell depleted host alloantigen or xenoantigen-bearing stimulators. To this culture will be added a gp39 antagonist, preferably an antibody or antibody fragment that specifically binds gp39 (CD154). Alternatively, the gp39 antagonist may comprise a soluble CD40 or soluble CD40 fusion protein, e.g., CD40Ig.

### Brief Description of the Drawings

Figure 1 shows the effect of anti-CD40L mAb treatment of donor T-cells in a primary MLR culture.
Figure 2A shows the effect of the addition of anti-CD40L (gp39) mAb on IL-2 production in primary MLR culture.
Figure 2B shows the effect of anti-CD40L mAb to gamma interferon production in a primary MLR culture.
Figure 3A shows the induction of anti-host alloantigen hyporesponsiveness by anti-CD40L mAb in secondary cultures is reversible by erogenous IL-2.
Figure 3B shows that donor T-cells exposed to anti-CD40 mAb in primary MLR culture have intact IL-2 responses in secondary culture.
Figure 4A shows the addition of anti-CD40L mAb to a primary MLR culture inhibits IL-1 production as measured in a secondary MLR culture.
Figure 4B shows that the addition of anti-CD40L mAb to a primary MLR culture inhibits gamma interferon production as measured in a secondary MLR culture.
Figure 5A shows that anti-CD40L mAb treatment of donor T-cells in an MLR culture markedly reduced *in vivo* GVHD capacity.
Figure 5B shows the effect of anti-CD40L treatment on mean body weight after transplantation.

### Detailed Description of the Invention

The following terms will be understood to have the following definitions:
Allogeneic Cell refers to a cell obtained from a different individual of the same species as the recipient.
Alloantigen refers to a cell obtained from a different individual of the same species as the recipient.
Xenogeneic cell refers to a cell obtained from a different species relative to another species, typically a transplant recipient. (For example, baboon T-cells would comprise xenogeneic cells if transplanted in a human recipient.)
Xenoantigen refers to an antigen expressed by a cell obtained from a different species relative to another species, typically a transplant recipient.
gp39 antagonist refers to a molecule that interferes with the gp39 (CD154) - CD40 interaction. A gp39 antagonist preferably will be an antibody directed against gp39 (e.g., a monoclonal antibody specific to human gp39), or a fragment or derivative thereof (e.g., Fab, F(ab)'₂ fragment, chimeric antibody, human antibody or humanized antibody). Also, gp39 antagonists include soluble forms of a fusion protein of a gp39 ligand (e.g., soluble CD40Ig) or pharmaceutical agents that interfere with gp39 - CD40 interaction.
gp39 or CD154 or CD40L or CD40CR is a molecule expressed on the surface of a T-cell that interacts with a molecule, CD40, identified on the surface of immature B-cell and mature B-cell lymphocytes that is involved in inducing B-cell proliferation. Specifically, the interaction with gp39 on T-cells with CD40 on B-cells plays a central role in activating B-cell responses to an antigen. Also, it has been discovered that gp39 plays a significant role in the response of T-cells to antigens, e.g., allo- and xenoantigens.
T-Cell non-responsiveness or T-cell tolerance in the present invention refers to the reduced immune response (graft-versus-host response) elicited by donor T-cells against allo- or xenoantigen bearing cells upon transplantation of these donor T-cells into a recipient after they have been contacted *ex vivo* with a gp39 antagonist (anti-gp39 antibody) and xeno- or alloantigen bearing cells.

As discussed, the present invention provides an alternative approach to the prevention of graft-versus-host disease upon transplantation of foreign donor T-cell containing compositions, e.g., allogeneic or xenogeneic bone marrow or peripheral blood cells.

It is known that a very small proportion of donor T-cells possess the capability to recognize host alloantigen (estimated to be less than 0.0%). The present invention seeks to eliminate this response (render such cells non-responsive or tolerized to alloantigen or xenoantigen) by functionally altering the population of T-cells with allo- or xenoantigen reactive capabilities.

It was hypothesized by the present inventors that this could potentially be accomplished by initiating a mixed lymphocyte reaction of donor T-cells and irradiated T-cell depleted host alloantigen or xenoantigen bearing stimulators, and adding to this mixed lymphocyte reaction culture a gp39 antagonist, in particular an anti-gp39 antibody. The hope was that this would interfere with gp39 - CD40 interactions *in vitro* (between donor T-cell and alloantigen or xenoantigen bearing cells), and render such cells tolerized or non-responsive to alloantigen or xenoantigen bearing cells upon transplantation into a transplant recipient. It was theorized that this would potentially be possible based on previous successful reports in the literature, including those of the present inventors, relating to inducing T-cell tolerance to allogeneic or xenogeneic tissue *in vivo* by the treatment of the transplantation recipient with gp39 antagonist (anti-gp39 antibody), alone or in combination with allogeneic or xenogeneic cells, prior, contemporaneous or subsequent to transplantation of xenogeneic or allogeneic tissue or organ. This *in vivo* approach has been demonstrated to be highly effective for indicating T-cell tolerance to various tissues or organs, e.g., bladder, skin, cardiac tissue, *et seq.*

However, it was unpredictable whether this methodology could be extended to the induction of T-cell tolerance or non-responsiveness *in vitro.* This outcome was not reasonably predictable because previous studies reported in the literature have demonstrated that there is no requirement that gp39 be present for the induction *of in vitro* T-cell activation. For example, Flavell and colleagues *(Nature,* 378:617-620 (1995)) have shown that T-cell receptor transgenic T-cells that were generically deficient in gp39 expression responded normally to antigen-presenting cells and antigen. This demonstrated that T-cell activation does not require gp39, and indeed can occur normally in the absence of gp39 *in vitro.*

Specifically, data reported by Grewal, J.S. et al, *Nature,* 378:617-620 (1995), "Impairment of antigen-specific T-cell priming in mice lacking CD40 ligand" demonstrated that there is no requirement that gp39 be present for short term *in vitro* activation of T-cells, and that allospecific cell T tolerance can be generated *in vitro* in the absence of gp39.

Therefore, quite unexpectedly it has been shown that T-cell tolerance or non-responsiveness of donor T-cells can be effectively induced *in vitro* by incubating such cells with a gp39 antagonist and recipient allogeneic or xenogeneic cells which are depleted of recipient T cells. This technique affords tremendous potential in the treatment of transplant recipients since it affords a highly efficient, non-invasive means of rendering transplanted T-cells contained in transplanted tissue or organ tolerized or non-responsive to recipient alloantigens or xenoantigens. Consequently, this transplanted tissue or organ, i.e., xenogeneic or allogeneic bone marrow should not elicit an adverse graft-versus-host response upon transplantation. Moreover, the fact that tolerance is induced *in vitro* is further advantageous as this treatment may be utilized in conjunction with other anti-rejection strategies, i.e., cyclosporine or other immunosuppressants. Also, it may be combined with anti-gp39 antibody administration (or other ligand) prior, concurrent or subsequent to transplantation.

In fact, the subject method may eliminate the need for other anti-rejection drugs, which given their immunosuppressant activity, may result in adverse side effects, e.g., increased risk of infection or cancer.

In the preferred embodiment, T-cells from the donor, e.g., an allogeneic or xenogeneic donor, will be cultured *in vitro* with recipient allogeneic or xenogeneic tissue which has been treated (e.g., irradiated) to deplete host T-cells. To this culture, an effective amount of a gp39 antagonist, typically an anti-gp39 antibody, will be added (e.g., 24-31 or 89-76 anti-human gp39 antibody disclosed in U.S. Patent No. GET 313 #) will be added. This culture will be maintained for a time sufficient to induce T-cell tolerance. Typically, this time will range from about 1-2 days to 30 days, more typically about 5 - 15 days, and most typically about 10 days.

After culturing, the donor T-cells can be tested to determine whether they elicit an anti-host allo- or xeno- response. Also, it can be determined whether such cells remain viable and otherwise elicit normal T-cell activity after treatment, e.g., IL-2 responses.

As shown in the Examples which follow, it has been found that donor T-cells when treated according to the invention exhibit markedly blunted anti-host xeno- or alloantigen responses, maintained viability, and further maintain intact IL-2 responses. Also, upon restimulation, donor T-cells maintained their anti-host alloantigen hyperresponsiveness.

It was also observed that in the primary MLR, the production of T-helper Type 1(Th 1) cytokines was markedly reduced. Similarly, in secondary restimulation cultures, Thl cytokine production was also markedly reduced.

Moreover, it was found that *in vivo* administration of equivalent numbers of control or anti-gp39 (CD154) monoclonal antibody treated donor T-cells had markedly different graft-versus-host disease properties. Specifically, recipients of three-fold higher number of donor T-cells in controls had a 50% actuarial survival rate as compared to 0% in controls. In other experiments, up to a 30-fold difference in graft-versus-host disease potentials were observed using anti-gp39 (CD154) monoclonal antibody treated T-cells. Based thereon, we have surprisingly concluded that donor T-cells can be effectively tolerized *ex vivo* by a mixed lymphocyte reaction. This should provide an important new approach for invoking donor T-cell tolerization to host cells and xenoantigens.

The method provides significant potential in the area of bone marrow or peripheral blood cell transplantation therapies. Bone marrow and stem cell transplantation is conventionally utilized for treatment of various diseases, such as leukemia and other diseases involving immune cell deficiencies. Moreover, bone marrow transplantation may afford benefits in the treatment of other diseases also, such as in the treatment of autoimmune diseases. However, a prevalent risk associated with conventional bone marrow transplantation therapy is the risk of eliciting a GVHD response. The subject method should reduce or even eliminate such risk and thereby extend the clinical indications for bone marrow transplantation therapies.

Essentially, these methods will comprise treating bone marrow or peripheral blood cells *ex vivo* as described above, and introduction of the treated bone marrow or peripheral blood cells into a recipient in need of such treatment, e.g., a cancer patient or person suffering from an autoimmune disease, in need of immune reconstitution because their own lymphoid cells have been depleted as a result of the disease or treatment of the disease (e.g., because of radiation treatment).

The present method may be combined with other anti-rejection treatments, e.g., *in vivo* infusion of immunosuppression agents such as methatrycide, cyclosporine A, steroids, or gp39 antagonist administration.

Ideally, the present method will provide for immune reconstitution in a recipient of the treated donor T-cells without eliciting any GVH response. However, in some instances, this therapy may need to be repeated if the transplanted tissue does not "take" in the transplant recipient. Alternatively, it may be necessary if the lymphoid system of the transplant recipient becomes impaired again as a result of disease or treatment or the disease, e.g., subsequent radiation treatment. In such cases, suitable donor T-cells will again be contacted *ex vivo* with anti-gp39 antibody and T-cell depleted allo-or xenoantigen bearing recipient cells, to induce T-cell tolerization, and then infused in the transplant recipient.

The invention is further illustrated by the following Examples which should not be construed as limiting. The contents of all references, patents and published patent applications throughout this application are incorporated by reference in their entirety.

### EXAMPLE 1

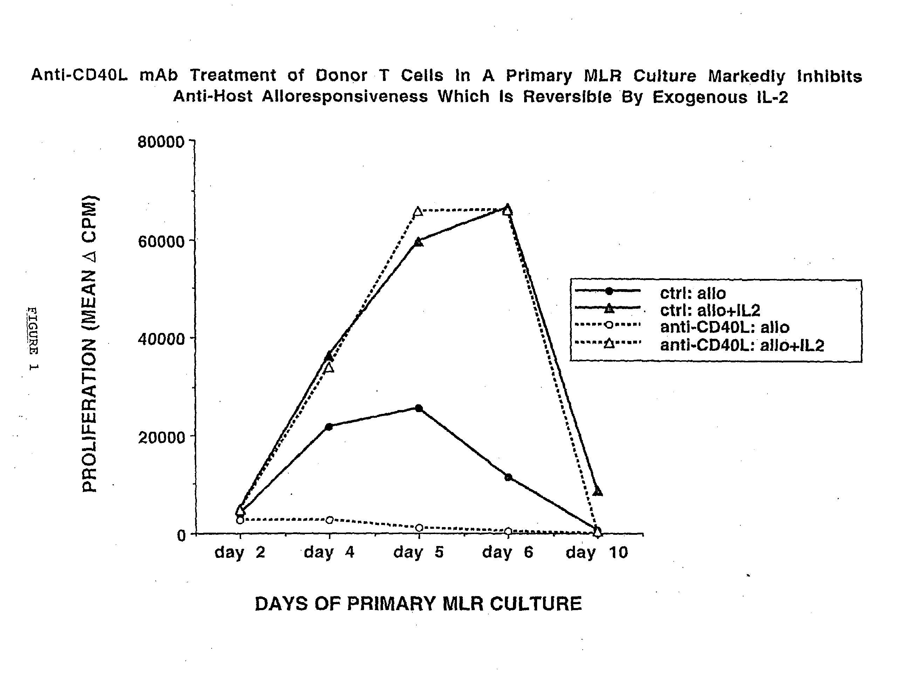

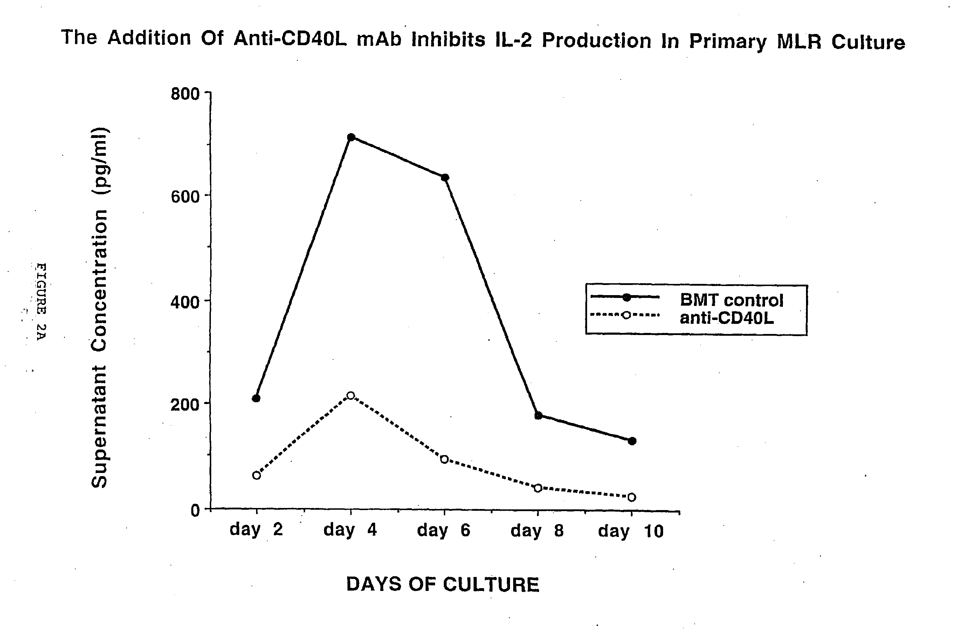

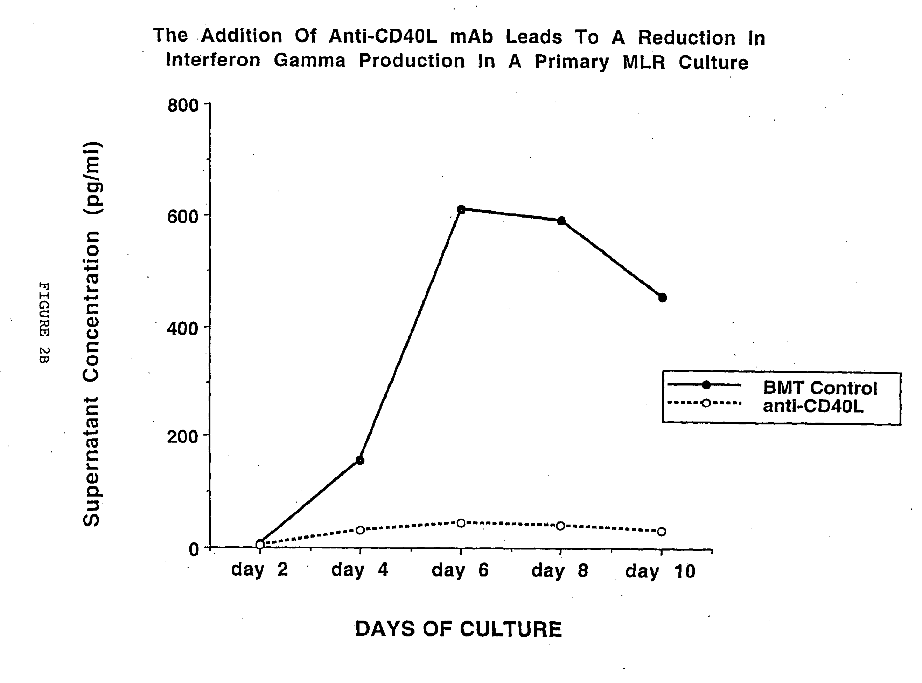

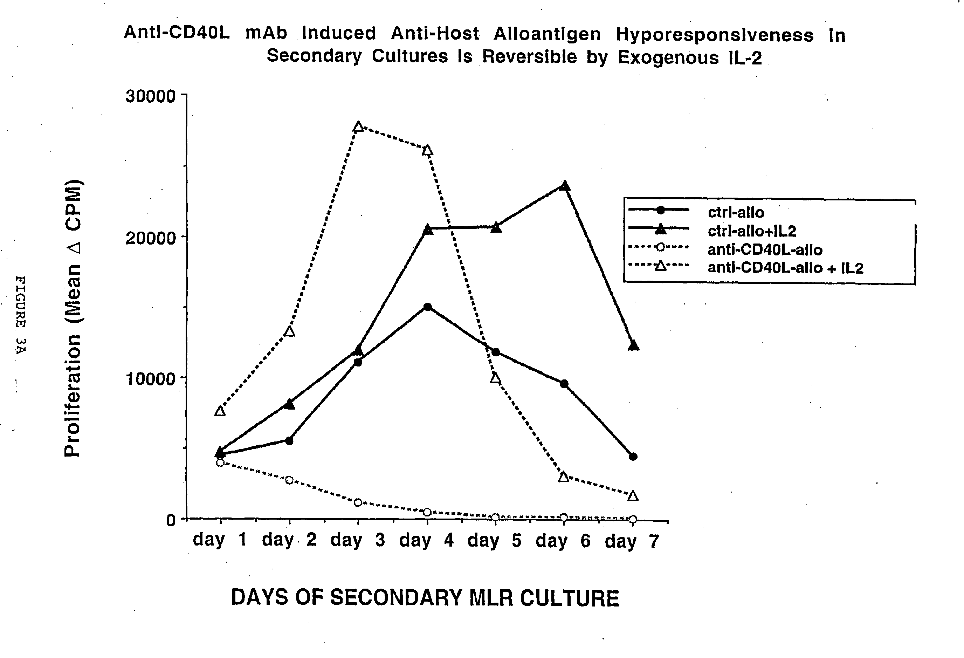

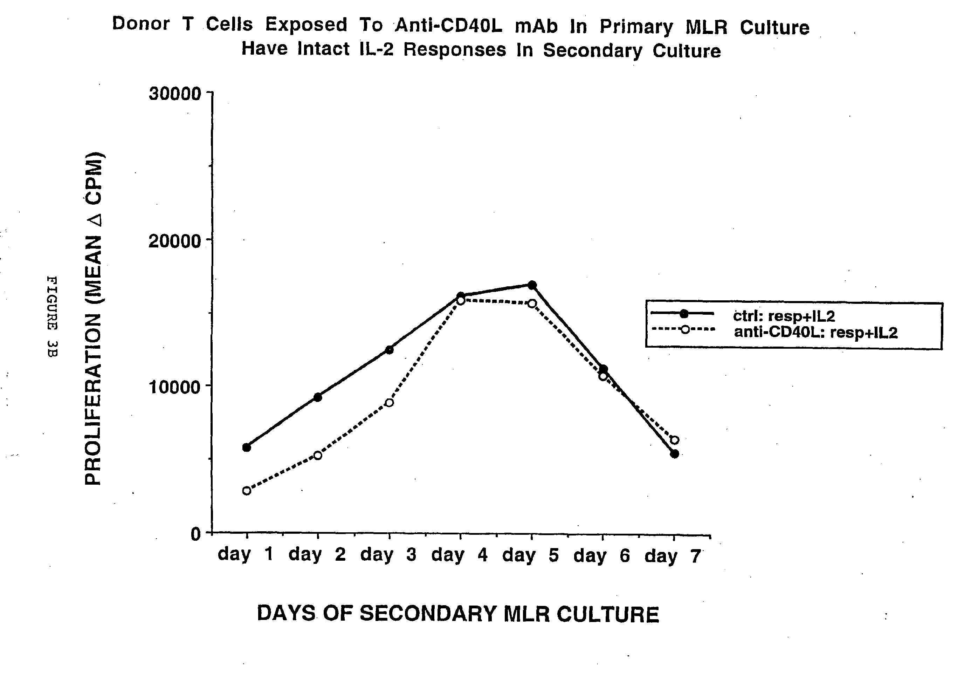

The results of a mixed lymphocyte reaction (MLR) between donor CD4+ lymph node T cells and MHC Class II disparate alloantigen bearing stimulator cells is shown in Figure 1. In this experiment, highly purified CD4+ lymph node T cells from C.H2^{bm12} were plated at a concentration of 0.5 x 10⁶ per ml final concentration in microtiter wells or in bulk culture in 24-well plates. Stimulator cells were C57BL/6 T cell depleted, irradiated spleen cells used at a final concentration of 1 x 10⁶ per ml. The MLR media consisted of 10% fetal calf serum, 5% supplements, and 2-ME. Anti-gp39 mAb was added at a final concentration of 50 micrograms per ml. Where indicated in Figure 1, IL-2 was added at a final concentration of 50 units per ml. Microtiter wells were pulsed with one microcurie per well of tritiated thymidine for an eighteen hour time period before harvesting. The mean Δ CPM (CPM of experimental - CPM of responders alone) are shown on the y axis and the days of primary MLR culture on the x axis. These data demonstrate a profound hyporesponsiveness in anti-gp39 mAb treated cultures which is reversible by addition of exogenous IL-2.

### EXAMPLE 2

Supernatants from vogue cultured cells from the experiment shown in Figure 1 were analyzed for the concentration of interleukin 2 (IL-2). These results are contained in Figure 1A. Supernatants were analyzed by ELISA (R&D Systems, Minneapolis, MN). Supernatant concentration in pg per ml were shown on the y axis and the days of MLR culture on the x axis. The additional of anti-gp39 mAb inhibited IL-2 production from donor T cells in a primary MLR culture.

### EXAMPLE 3

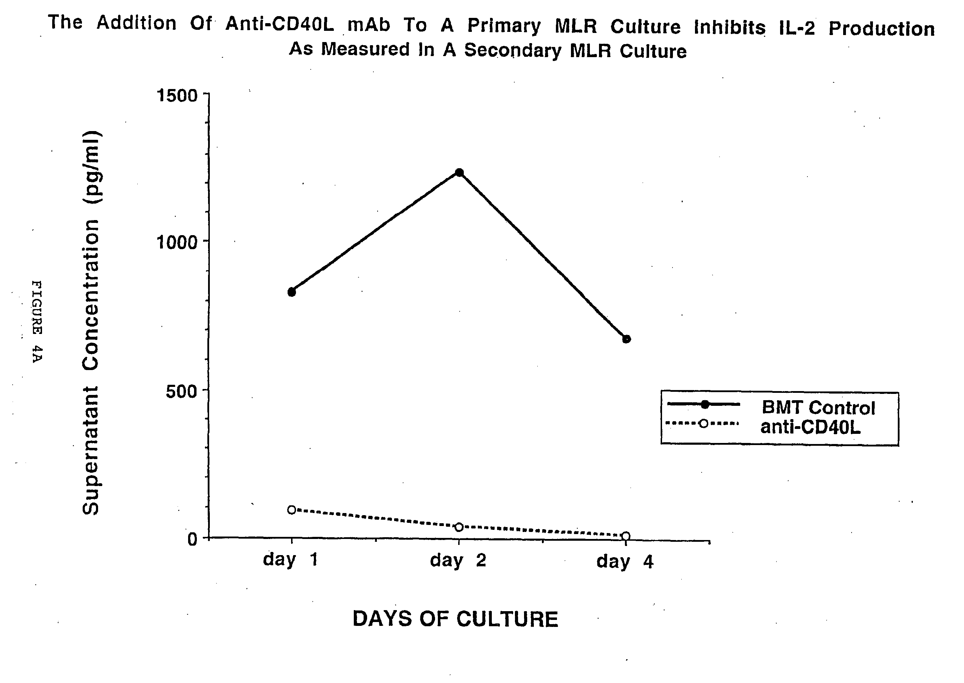

The supernatant concentration of interferon gamma was analyzed by ELISA in the same cultures used in the experiment the results of which are contained in Figure 2A. These results are contained in Figure 2B. It can be seen that the addition of anti-gp39 mAb was observed to lead to a profound reduction of interferon gamma production and a primarily MLR culture.

### EXAMPLE 4

At the end of the ten day cell culture period, cells were phenotyped by two color flow cytometry. As can be seen in Table 1 (after examples), the addition of anti-gp39 mAb did not prevent T cell activation as evidenced by the high levels of CD25, OX40, CTLA-4, B7-1 and B7-2. The addition of anti-gp39 mAb, however, did inhibit the conversion of naive T cells to effector T cells as demonstrated by the high levels of L-selectin, ICAM-1 and low levels of CD45. Cells in the treated culture were not undergoing apoptosis that is evidenced by the relatively lower positivity for 7-AAD.

### EXAMPLE 5

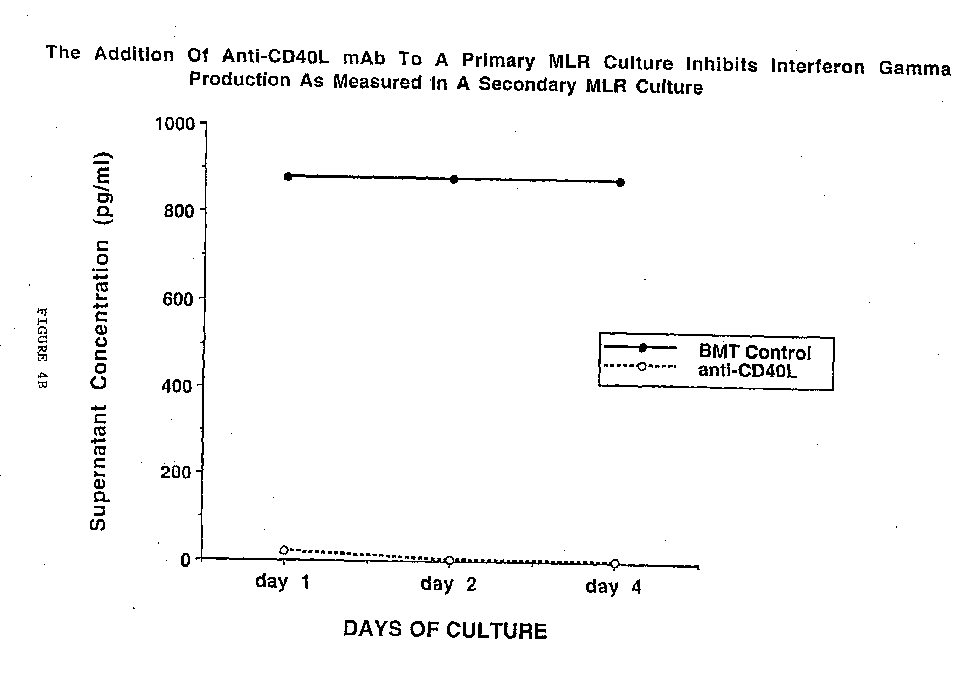

At the end of the primary MLR culture, cells were washed and replated at a concentration of 3 × 10⁴ per 96 well plate. To each well, irradiated splenocytes from C57BL6 mice were added at a concentration of 10⁵ cells per well. These results are contained in Figure 3A. Where indicated, IL-2 is added at a final concentration of 50 units per ml. The media consisted of 10% fetal calf serum, 5% supplements, 2-ME. Microtiter wells were labeled with one microcurie per well at the indicated times for a period of eighteen hours prior to harvesting. On the y axis are the mean proliferation values (Δ CPM) and on the x-axis are the days of secondary MLR culture. As can be seen from the results in Figure 3H, donor T cells exposed to anti-gp39 mAb in primary but not secondary culture retained alloantigen specific hyperresponsiveness in the secondary culture. This was reversible by the addition of exogenous IL-2 in the secondary culture alone.

### EXAMPLE 6

In separate cultures, donor T cells from control treated cultures or anti-gp39 mAb treated primary MLR cultures were exposed to exogenous IL-2 at 50 units per ml final concentration. These results are contained in Figure 3b. It can be seen that there is an equivalent response of donor T cells from control treated as compared to anti-gp39 mAb treated primary MLR cultures as assessed under the secondary conditions.

### EXAMPLE 7

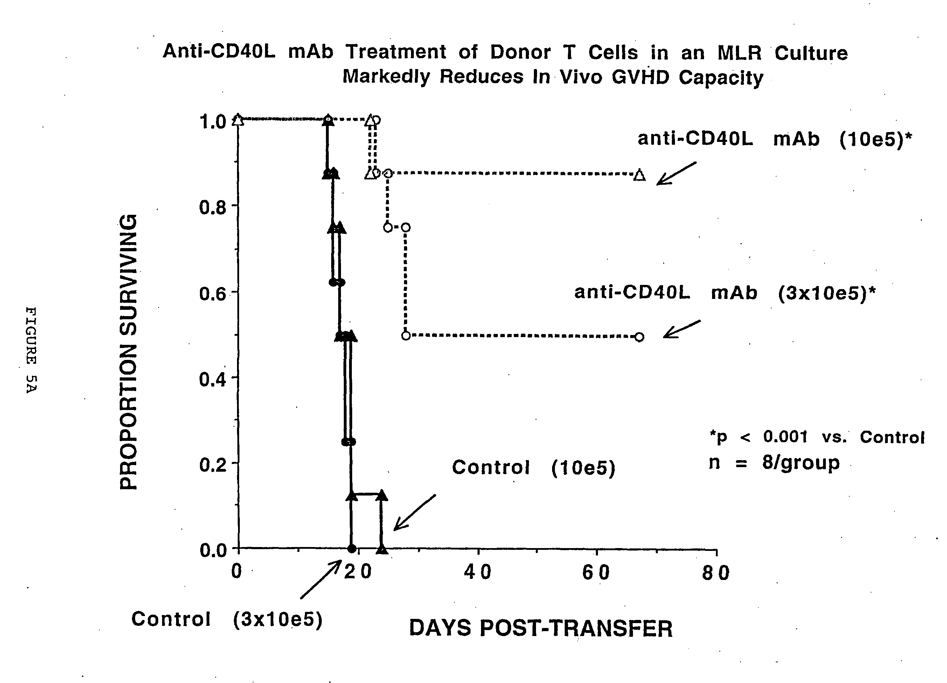

Supernatants obtained from the secondary MLR bulk cultures were tested by ELISA for the production of IL-2 (Figure 4A) or interferon gamma (Figure 4B) as measured in a secondary MLR culture. It can be seen therefore that donor T cells exposed to anti-gp39 mAb in primary but not secondary MLR cultures continue to have a markedly low supernatant concentration of IL-2 (Figure 4A) and interferon gamma (Figure 4B).

### EXAMPLE 8

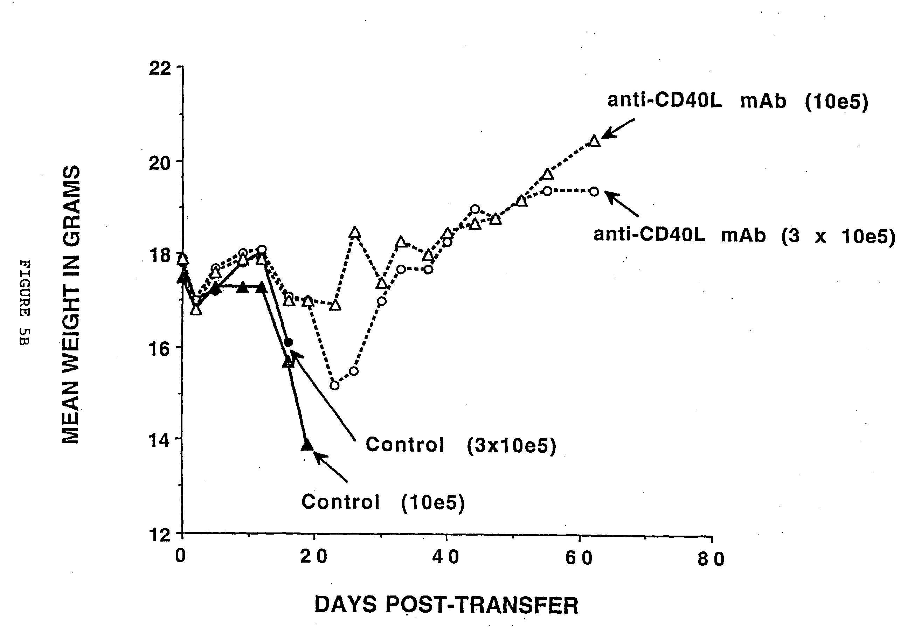

At the end of the primary MLR culture, donor T cells were administrated to sublethally irradiated (600 cGray total body irradiation) C57BL/6 recipients. Two cell doses were tested (10⁵ or 3 × 10⁵). These results are contained in Figure 5. It can be seen from Figure 5 that recipients of controlled cultured cells at either cell dose uniformly succumb to lethal GVHD prior to four weeks post transplantation. In contrast, recipients of 10⁵ donor T cells exposed to anti-CD40L mAb *ex vivo* had an 88% survival rate. Recipients of 3 x 10⁵ donor T cells exposed to anti-CD40L mAb had a survival rate of 50% at time periods greater than two months post transfer. When compared to recipients of donor T cells obtained from control cultures, the actuarial survival rates of recipients of an equal number of donor T cells exposed to anti-CD40L mAb treated was significantly (p <0.001) higher at both cell doses.

### EXAMPLE 9

The animals in the experiment, the results of which are contained in Figure 5A were monitored for evidence of GVHD by mean weight curves. These results are contained in Figure 5B. It can be seen therefore that recipients of control cells had a marked decrease in mean weight curves (y-axis) beginning 2.5 weeks post transfer which resulted in GVHD lethality prior to 4 weeks post transfer. In contrast, recipients of anti-gp39 mAb treated cells had weight curves that exceeded their pre-transfer mean body weights. Also, recipients of 10⁵ or 3 x 10⁵ cells from control cultures had a marked reduction in mean body weight, consistent with a GVH reaction. This demonstrated that GVHD lethality was inhibited by treatment with anti-gp39 mAb.

Moreover, in other experiments, up to a 30-fold reduction in GVHD mortality has been observed in recipients receiving anti-gp39 mAb treated cultures as compared to controls.

### EXAMPLE 10

The *in vivo* expansion of donor alloreactive T cells was examined. Donor T cells from the experiment shown in Figures 1 - 5 and Table 1 and 2 were infused into mice with severe combined immune deficiency. These recipients were disparate with the donor at MHC class I + class II loci. On day 6 post transfer, mice were given a continuous intravenous infusion of 1 ml per hour (representing about 1/4-1/3 of the animals total body water per hour) of fluids. The thoracic duct lymphatics were cannulated and thoracic duct lymphocytes were collected during an overnight collection procedure. Approximately 1 ml per hour per animal is collected prior to death. The number of CD4+ T cells produced per day can then be quantified. It can be seen that the recipients of control cultured cells which produced an average of 2 x 10⁶ CD4+ T cells per ml of thoracic duct effluent. By contrast, recipients of anti-gp39 mAb treated cultures produced only 0.3 x 10⁶ CD4+ T cells per ml representing an approximate 7-fold reduction in the generation of alloreactive T cells *in vivo.* These data provide additional evidence that anti-gp39 mAb reduces the capacity of donor T cells *in vivo* to mediate lethal GVHD.

In summary, the results of the above experiments provide conclusive evidence that anti-gp39 mAb markedly reduced GVHD capacity *in vivo.* This represents a new methodology for tolerizing donor T cells to host antigens or alloantigens *ex vivo* as a means of preventing lethal GVHD *in* vivo.

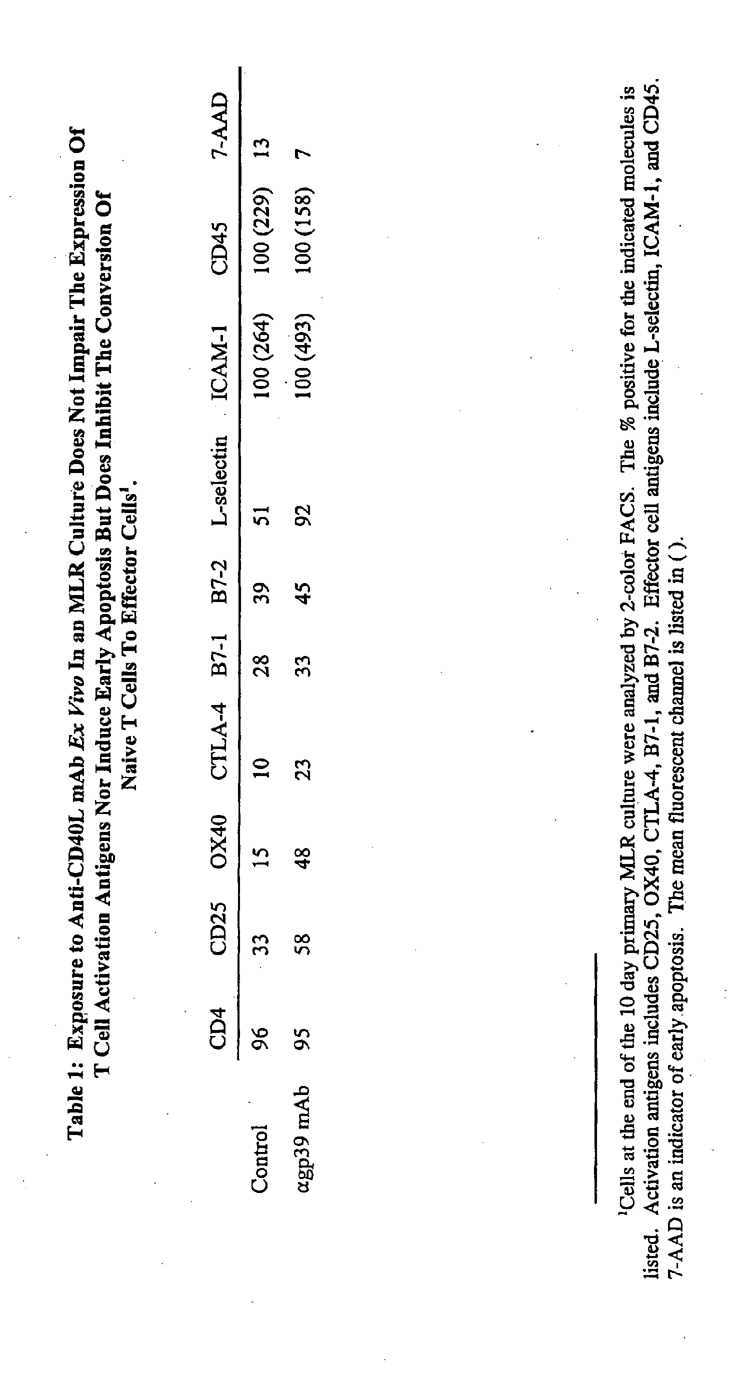

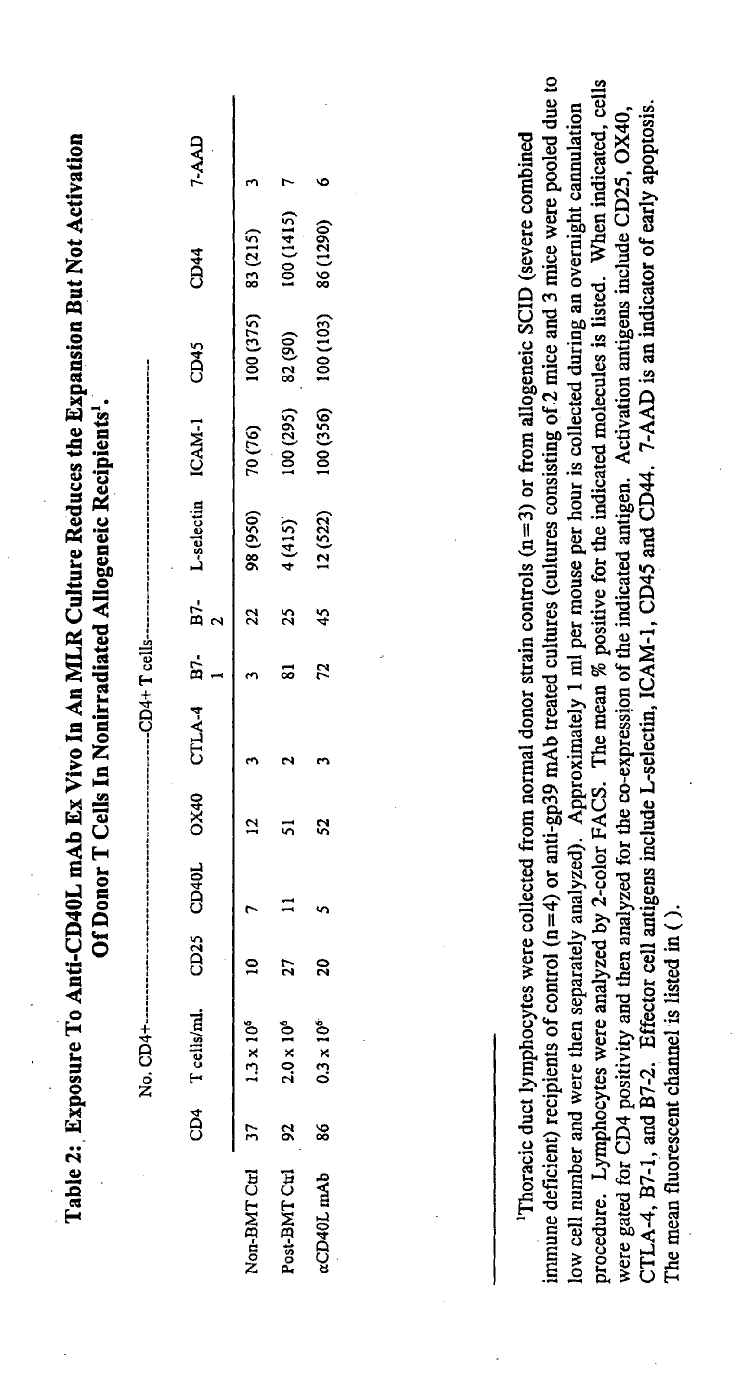

Those skilled in the art will recognize, or be able to ascertain using no more than routine experimentation, many equivalents to the specific embodiments of the invention described herein. Such equivalents are intended to be encompassed by the following claims.

## Claims

1. A method for inducing T-cell tolerance or non-responsiveness of donor T-cells to desired alloantigen or xenoantigen bearing cells *in vitro* comprising the following:
(i) providing a culture containing donor tissue containing donor T-cells;
(ii) producing a mixed lymphocyte reaction culture by adding to said donor T-cell culture alloantigen or xenoantigen-bearing cells;
(iii) adding to the resultant mixed lymphocyte culture a gp39 antagonist; and
(iv) maintaining these cells in culture for a sufficient time to render the donor T-cells substantially non-responsiveness to said alloantigen or xenoantigen bearing cells.

2. The method of Claim 1, wherein the tissue containing donor T-cells is donor bone marrow or peripheral blood cells.

3. The method of Claim 1, wherein the gp39 antagonist is selected from the group consisting of an anti-gp39 antibody, soluble CD40 and soluble CD40 fusion protein.

4. The method of Claim 3, wherein the gp39 antagonist is an anti-gp39 antibody.

5. The method of Claim 4, wherein said anti-gp39 antibody is an anti-human gp39 monoclonal antibody.

6. The method of Claim 1, wherein the donor T-cells are cultured with said gp39 antagonist for a time ranging from about 1 to 30 days.

7. The method of Claim 6, wherein said time ranges from 5 to 15 days.

8. The method of Claim 1, wherein the alloantigen or xenoantigen bearing cells comprise cells or tissue obtained from a potential transplant recipient that has been treated to deplete recipient T-cells.

9. The method of Claim 8, wherein T-cell depletion is effected by irradiation.

10. The method of Claim 1, wherein the donor T-cells are transplanted into a recipient in need of such transplantation.

11. The method of Claim 10, wherein the recipient is in need of immune reconstitution as a result of disease or disease treatment.

12. The method of Claim 11, wherein said disease is cancer or autoimmune disease.
